# EUROPEAN PATENT APPLICATION

(11) **EP 3 000 891 A1**
(43) Date of publication of application: **30.03.2016**
(21) Application number: 14461572.1
(22) Date of filing: 26.09.2014
(51) Int. Cl.: C12P 3/00, C12N 1/00

(54) **Microbiological consortium for the purification of biogas**

(71) Applicant: Politechnika Lódzka, 90-924 Lódz (PL)
(72) Inventor: Zieminski, Krzysztof, 93-355 Lódz (PL)
(74) Representative: Rumpel, Alicja

(57) **Abstract**

Biogas purifying microbiological consortium in the presence of oxygen forms of nitrogen, containing α-Proteobacteriae , β-Proteobacteriae and γ-Proteobacteriae.

Preferably the microbiological consortium contains bacteria of the following species: Citrobacter werkmanii , Denitratisoma oestradiolicum , Denitrobacter permanent , Limnohabitans parvus , Luteibacter anthropi , Methylotenera versatilis , Oxalobacter vibrioformis , Propionivibrio dicarboxylicus , Ralstonia insidiosa , Rhodanobacter fulvus , Rhodanobacter ginsenosidimutans , Rhodanobacter lindaniclasticus , Thermomonas dokdonensis , Thermomonas Fusa , Thiobacillus thioparus , Thiobacillus thiophilus , Thiomonas intermedia , Thiomonas perometabolis , Vogesella perlucida , Ralstonia detusculanense , Aminobacter aminovorans and Comamonas composti.

## Description

The invention deals with biogas purifying microbiological consortium in the presence of oxygen forms of nitrogen in a biofilter with biological sediment.

Biogas is generated by anaerobic bacteria, causing degradation of organic substances. Biogas mixture usually contains approximately 55-85% of methane (CH₄), 30-45% of carbon dioxide CO₂), 0.01-5% of hydrogen sulphide (H₂S) plus low concentrations of nitrogen, oxygen and water steam. The main cause of reduced fuel calorific value is the presence of contaminations, especially of H₂S. Biogas purification from unnecessary substances considerably increases its calorific value. The need of biogas desulphurisation is also associated with the necessity to eliminate negative effects of H₂S on biogas combustion systems, protect engines of electrical power plants and prevent boilers and boiler burners from corrosion. Sulphated biogas combustion results in enhanced emissions of sulphur dioxide, the gas which is responsible for acid rains. At the same time, there is a need to reduce emissions of H₂S as a component with strong toxic effects, highly detrimental for human and environmental health.

Both in biogas production and use, technologies are preferred with no burdens for the natural environment. However, biogas treatment to achieve its composition, which would be suitable for gas networks is a cumbersome and expensive task with the use of conventional methods, based mainly on active carbon sorption properties. For the above-mentioned reasons, it is necessary to develop biological methods of biogas purification, as an alternative for chemical processes. They provide an attractive option for the low capital outlays and the lack of negative effects on the natural environment. The main assumptions of the biotechnological methods include having appropriate, specially selected microorganisms plus the knowledge in which they may effectively be used. Actually, the key problem is the lack of highly effective methods of biological biogas purification on industrial scale. Another problem, associated with the application of biological methods is the presence of elementary sulphur in the installation on which desulphurisation is performed. Sulphur compounds are oxidised to elementary sulphur which is then deposited in the system and has to be regularly removed.

Studies, carried out at research & development usnits are usually based on single microorganism strains. It is highly probable that these microorganisms will not survive in industrial conditions, where they will have to compete with the natural microflora. Moreover, one should remember that the biogas purification process in industrial conditions should not be carried out by a single bacterial strain.

Biogas with high hydrogen sulphide concentration may successfully be cleaned by biological methods in systems of columns with differentiated packing levels, on the surface of which a biological film is formed of the gas degrading bacteria. Such a type of biofilm usually transforms the absorbed hydrogen sulphide into elementary sulphur or sulphates, depending on the volume of available oxygen. In as much as from the technological point of view, the knowledge of biogas purification processes in such systems is fairly advanced, the structure and dynamics of such biocenosis functionality, perceived from the microbiological perspective is still a kind of enigma. It often happens that the value of microbiological biodiversity in such systems is associated with the the spatial layout of bacteria in a column and may thus differ at its various levels. Following the reports of Calvo-Bado et al. (2003), Roy et al. (2009) and Sakano et al. (2002), biodiversity in biogas purifying columns is usually higher in their inlet parts. It is explained by a higher diversity of available nutrition sources, in particular, easily degradable and, additionally, in non toxic concentrations. However, when toxic compounds in high concentrations are delivered to the column inlet, the situation is quite different. Then the biodiversity of bacteria at the column inlet is lower, probably due to the specialisation of certain microorganism groups (Bayle et al., 2009; Van der Gast et al., 2008).

The determinants of bacteriological variability include physical factors (pH, temperature, oxygenation), chemical factors (culture medium composition, the presence of toxic compounds), as well as the ecological relationships in studied biocenosis (the origin, resulting from the composition of bacterial inoculum and positive and negative interactions within the system). It should be emphasised that the very conditions of the process procedure and the inoculum composition are satisfactory to significantly modify the structure of primary biocenosis, without the presence of toxic factors / contaminations (Acbrol and Malhautier, 2011; Ding et al., 2008; Sei et al., 2004). In case of biogas purifying systems, the very transition of inoculum into the column causes its drastic quantitative-qualitatitve depletion during the adaptation phase (Maestre et al., 2010). As a rule, such diversity depletion is regarded as a specialisation of biocenosis by selection of the best adapted microorganisms , exclusion by competition, the toxic effects of contaminations or reduction of the pool of available nutrition sources (Eichner et al., 1999; Steele et al., 2005; Konopka et al., 2007,

Patent description No. WO2012061933 presents a system of H₂S eliminating bioreactor, where a psychotrophic anaerobic bioreactor was applied, operating in microaerophilic conditions, where the microconsortium is built by the bacteria of an active anaerobic sediment. In this purification process, a special reaction provides an elementary sulphur product, which is then converted into deposits. What is more, oxygen injection dissolves biogas. Patent description No. EP1604727 depicts a biogas desulphurisation method in presence of sulphate and iron ions by means of halotolerant microorganisms. In this purification process, a special reaction provides a product in the form of elementary sulphur. Patent description No. WO2008131034 presents a bioscrubber system to remove H₂S with hydrogen sulphide oxidising bacteria, acquired from hot springs. H₂S is oxidised into sulphur and SO₄²⁻. In result of the process, pH level decreases, what has then to be compensated.

The microbiological consortium, according to this invention, was acquired from a natural environment, rich in sulphur. The consortium was immobilised on a porous, filtration packing of the column.The microbiological consortium includes bacteria of α-Proteobacteria, β-Proteobacteria and γ-Proteobacteria, belonging to the following orders: Burkholderiales, Xanthomonodales, Rhodocyclales, Hydrogenophilales, Rhizobiales, Sphingomonodales, Enterobacteriales, Methylophilales , Caulobacteriales , Neisseriales , Rhodospirrales , Pseudomonadales , Oceanospirillales , Methylococcales, belonging to the following genera: Rhodanobacter , Thiomonas , Ralstonia , Thermomonas , Simplicispira , Thiobacillus , Propionivibrio , Sphingomonas , Oxalobacter , Denitratisoma , Thermomonas , Parvibaculum , Simplicispira , Hylemonella , Comamonas , Citrobacter , Pedomicrobium , Methylotenera , Alcanivorax , Acinetobacter , Aminobacter , Methylomicrobium , Methylomonas , Acidovorax , Azospira , Pseudomonas , Candidatus Blochmannia and Sphingopyxis.

Preferably the microbiological consortium contains bacteria of the following species: Citrobacter werkmanii , Denitratisoma oestradiolicum , Denitrobacter permanent , Limnohabitans parvus , Luteibacter anthropi , Methylotenera versatilis , Oxalobacter vibrioformis , Propionivibrio dicarboxylicus , Ralstonia insidiosa , Rhodanobacter fulvus , Rhodanobacter ginsenosidimutans , Rhodanobacter lindaniclasticus , Thermomonas dokdonensis , Thermomonas Fusa , Thiobacillus thioparus , Thiobacillus thiophilus , Thiomonas intermedia , Thiomonas perometabolis , Vogesella perlucida , Ralstonia detusculanense , Aminobacter aminovorans and Comamonas composti.

Moreover, the biological consortium may also include bacteria, belonging to the following classes: Actinobacteria, Sphingobacteria, Verrucomicrobiae, Holophagae, Planctomycetia, Verrucomicrobiae, Clostridia, ε-Proteobacteria, Δ-Proteobacteria, Flavobacteria, Cyanobacteria, Bacteriodetes which belong to the following orders: Actinomycetales , Sphingobacteriales , Holophagales , Bacteroidales , Verrucomicrobiales , Gemmatales , Campylobacteriales , Desulfurellales , Flavobacteriales , Stigonematales, belonging to the following genera: Geothrix , Rhodocuccus , Prosthecobacter , Singulisphaera , Cellulomonas , Arthrobacter , Desulfurella , Segetibacter , Chryseobacterium, belonging to the following species: Rhodothermus clarus , Arthrobacter halodurans , Arthrobacter psychrolactophilus , Arthrobacter stackebrandtii, Chryseobacterium soli and Segetibacter aerophilus.

The advantage of this invention is 97-100% H₂S reduction in industrial conditions, allowing to achieve biogas parameters comparable with the natural gas parameters. Another advantage of this invention is a minimised sulphur production in the biogas purification process and a limited deposit volumes in the installation, on which the process is carried out. The process does not reduce pH, either, what eliminates the need for later pH correction. The purified biogas is not dissolved with oxygen, what allows avoiding the danger of explosive oxygen and biogas mixture formation, while also reducing the costs of the installation maintenance.

The subject of the invention has been explained in a more detail in the application examples.

### Example 1: A system for biogas desulphurisation and an analysis of microbiological consortium.

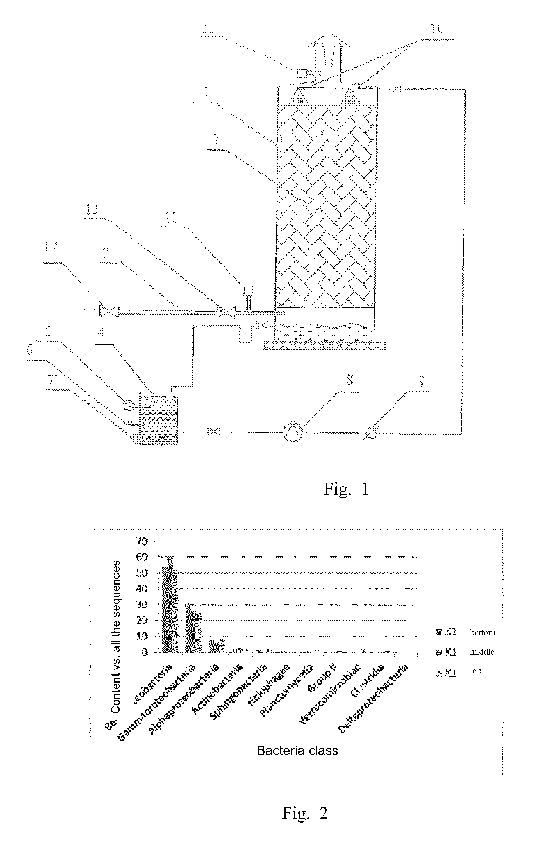

This invention employed a system, based on a sprinkled biofilter, working as an element of the industrial installation. Contaminated gas was delivered from the bottom of the system, while a recirculating culture medium sprinkled the column packing from the top in a volume of 30 l/h. The microorganisms, making the consortium of microorganisms according to this invention, were isolated from natural environments, rich in sulphur. The microorganisms were immobilised on a carrier, providing the column packing. The process was carried out in anaerobic conditions. The installation for biogas purification has been illustrated in the drawing, where fig. 1 presents its structure. Biogas, produced in the process of methane fermentation, was pumped under 300 ppm pressure into the pipeline (3) and then to the biological desulphurisation system. The flowing gas volume was controlled with a flow control (13). H₂S measurement and recording, both at inlet and outlet from the biofilter, was performed by H₂S concentration sensors (11). The biofilter, consisting of a porous filtration material, in form of plastic blocks (2), allowing the formation of specific biofilm with activity level, ensuring bioga purification, was placed in the column (1). The bio filter structure was sprinkled from top (10) with the culture medium in a volume of 30 l/h. The culture medium, dripping through the biofilter down to the column bottom, was drained into the tank (4). The culture medium was then returned to the system with a pump (8), flowing through a flowmeter (9) up to the sprinklers (10). The culture medium tank (4) had a heater (7) with a temperature sensor (5) to maintain constant temperature in the tank, as well as a culture medium level sensor (6). Biogas flow to the installation was cut off by the valve (12). The column operated for 45 days. Desulphurising efficiency was 98-99%.

Fig. 1. Installation for biogas purification

The microbiological consortium at three column levels (bottom, middle level, top) was characterised after installation and after 45 days of operation by sequencing the products of PCR amplification.

DNA from samples was isolated by means of a Bead-Beat Micro Gravity DNA isolation kit of A&A Biotechnology (ref. No. 106-20). DNA quantification was carried out with a Quant-iT PicoGreen dsDNA Assay kit of Molecular Probes (ref. No. P11496) by fluorescence measurement. DNA amplification reactions for sequencing purposes were carried out acc. to the description, presented in the publication of Caporaso et al. (2012). The sequencing was done with a MiSeq v2 2x250nt Reagent Kit of iLLUMINA (ref. No. 15033625), using the starters, presented in Caporaso et al. (2012) The sequencing results were automatically analysed on a MiSeq device of Illumina, using the MiSeq Reporter (MSR) software v2.4, 16S Metagenomics protocol. The analysis consisted of three stages: i) automatic demultiplexing of samples, ii) generating of fastq files with raw recordings, iii) classification of paired-end readouts in particular taxonomic categories. The 16S Metagenomics protocol ensured a classification of readouts to the species level, on the basis of Greengenes v13_5 reference sequence data, modified by the Illumina Company. The preparation of the reference database of reference sequences included: i) defiltration of the sequences below 250 base pairs; ii) defiltration of the sequences with more than 50 degenerated bases (M, R, W, S, Y, K, V, H, D, B, N); iii) defiltration of incompletely classified sequences (no classification to the level of genus or species).

Fig. 2. A list of percentage shares of particular bacteria classes at the three levels of the column packing - the bottom (blue colour), the middle level (red colour) and the top (green colour) at column operation starting point.

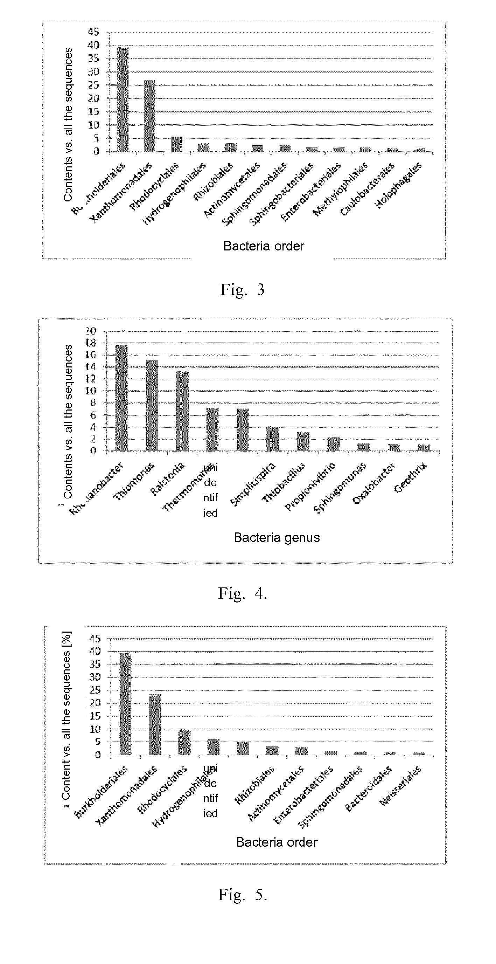

Fig. 3. Percentage share of particular bacteria orders in the bottom part of the column packing at column operation starting point.

Fig. 4. Percentage share of particular bacteria genera in the bottom part of the column packing at column operation starting point.

Fig. 5. Percentage share of particular bacteria orders in the middle part of the column packing at column operation starting point.

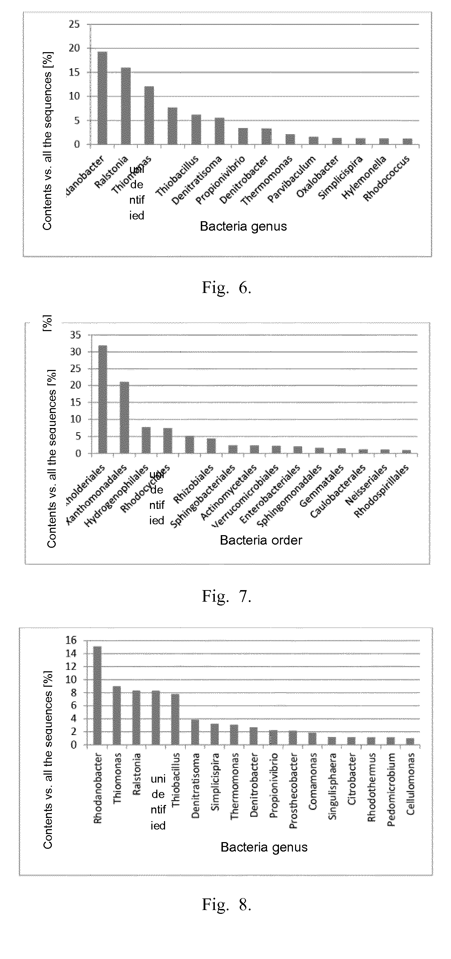

Fig. 6. Percentage share of particular bacteria genera in the middle part of the column packing at at column operation starting point.

Fig. 7. Percentage share of particular bacteria orders in the upper part of the column packing at column operation starting point.

Fig. 8. Percentage shares of particular bacteria genera in the upper part of the column packing at column operation starting point.

**Table 1. List of species in the highest number in particular parts of the column packing at column operation starting point.**

| bacteria species | Localisation in column | | |
|---|---|---|---|
| | the bottom | the middle level | the top |
| unidentified | 35.68 | 33.81 | 37.81 |
| Citrobacter werkmanii | 0.6 | 0.15 | 0.76 |
| Denitratisoma oestradiolicum | 1.98 | 5.54 | 3.84 |
| Denitrobacter permanens | 0.19 | 0.94 | 0.64 |
| Limnohabitans parvus | 0.86 | 0.24 | 0.8 |
| Luteibacter anthropi | 0.59 | 0.69 | 0.55 |
| Methylotenera versatilis | 0.65 | 0.36 | 0.31 |
| Oxalobacter vibrioformis | 1.14 | 1.33 | 0.9 |
| Propionivibrio dicarboxylicus | 3.25 | 3.41 | 2.23 |
| Ralstonia insidiosa | 12.6 | 15.07 | 7.82 |
| Rhodanobacter fulvus | 1.02 | 0.47 | 0.97 |
| Rhodanobacter ginsenosidimutans | 2.33 | 1.4 | 2.16 |
| Rhodanobacter lindaniclasticus | 4.08 | 5.15 | 3.38 |
| Rhodothermus clarus | 0.26 | 0.04 | 1.13 |
| Thermomonas dokdonensis | 2.41 | 0.69 | 0.86 |
| Thermomonas fusca | 1.53 | 0.61 | 0.97 |
| Thiobacillus thioparus | 0.14 | 0.08 | 1.17 |
| Thiobacillus thiophilus | 2.58 | 5.44 | 4.97 |
| Thiomonas intermedia | 14.14 | 10.91 | 8.41 |
| Thiomonas perometabolis | 0.65 | 0.96 | 0.44 |
| Vogesella perlucida | 0.56 | 0.69 | 0.84 |

The microbiological consortium was also characterised after 45 days of the column operation.

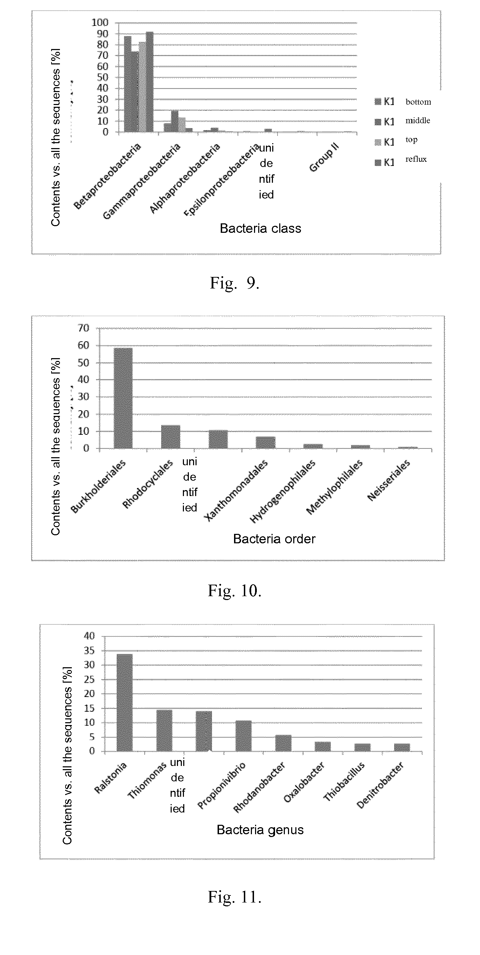

Fig. 9. A list of percentage shares of particular bacteria classes at the three levels of the column packing - the bottom (blue colour), the middle level (the red colour) and the top (green colour) and in the reflux (violet colour) after 45 days of column operation.

Fig. 10. Percentage share of particular bacterial orders in the bottom part of the column packing after 45 days of column operation.

Fig. 11. Percentage share of particular bacteria genera in the bottom part of the column packing after 45 days of column operation.

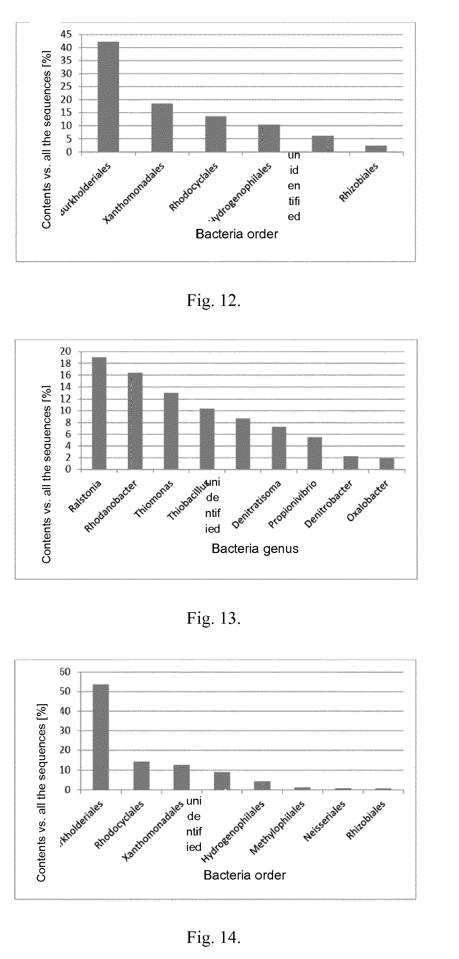

Fig. 12. Percentage share of particular bacteria orders in the middle part of the column packing after 45 days of column operation.

Fig. 13. Percentage share of particular bacteria genera in the middle part of the column packing after 45 days of column operation.

Fig. 14. Percentage share of particular bacteria orders in the upper part of the column packing after 45 days of column operation.

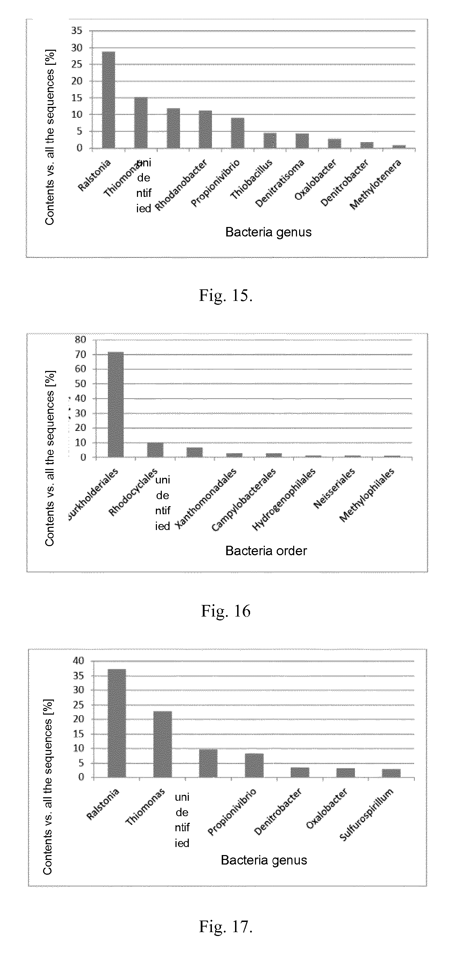

Fig. 15. Percentage share of particular bacteria genera in the upper part of the column packing after 45 days of column operation.

Fig. 16. Percentage share of particular bacteria orders in the column reflux after 45 days of column operation.

Fig. 17. Percentage share of particular bacteria genera in the column reflux after 45 days of column operation.

**Table 1. List of species in the highest number in particular parts of the column packing after 45 days of column operation.**

| bacteria species | Localisation in column | | | |
|---|---|---|---|---|
| | the bottom | the middle level | the top | reflux |
| unidentified | 23.32 | 26.36 | 23.99 | 20.19 |
| Denitratisoma oestradiolicum | 1.59 | 1.92 | 4.27 | 1 |
| Denitrobacter permanens | 1.02 | 0.79 | 0.6 | 1.07 |
| Methylotenera versatilis | 1.24 | 0.58 | 0.84 | 0.58 |
| Oxalobacter vibrioformis | 3.28 | 1.92 | 2.81 | 3.12 |
| Propionivibrio dicarboxylicus | 10.74 | 5.48 | 8.94 | 8.32 |
| Ralstonia detusculanense | 1.57 | 0.9 | 1.18 | 1.14 |
| Ralstonia insidiosa | 31.84 | 17.85 | 27.26 | 35.69 |
| Rhodanobacter lindaniclasticus | 2.34 | 6.47 | 4.93 | 0.81 |
| Thiobacillus thiophilus | 2.38 | 9.39 | 4.18 | 0.89 |
| Thiomonas intermedia | 13.4 | 11.9 | 14.23 | 21.08 |
| Thiomonas perometabolis | 0.92 | 0.94 | 0.8 | 1.37 |

### Example 2: Microbiological consortium at the column packing on a semi-technical scale at the sewage treatment plant 1.

A biogas desulphurisation system was applied on a semi-technical scale at the sewage treatment plant 1, servicing 820,000 inhabitants. The actual sewage treatment plant capacities are 819,000 cubic meters / day (preliminary mechanical), 450,000 cubic meters / day (mechanical) and 215,000 cubic meters / day (biological - biogen removal capacity). The sewage treatment plant has got a biological system for nitrogen and phosphorus removal, based on an active sediment. The process of biological purification takes place at rectangular chambers of active sediment, while the separation of sewage from the sewage-sediment mixture is performed in 7 rectangular secondary sedimentation tanks. The calculated total time of sewage retention for the flow of 215.3 cubic meters / day is 15.5 hours, including 1.9 hours in the anaerobic chamber, 6.0 hours in the low-oxygen chamber and 7.6 hours in the oxygen chamber. Sediment stabilisation is continued in the process of mesophilic fermentation. The process is arranged in four closed chambers of 10,000 cubic meters each. The energy is produced at two combined plants: the boiler room and the power and heating plant. The fuel is biogas, obtained from the fermentation process of sewage sediments.

Desulphurisation efficiency was 99.4%. The microbiological consortium at three column levels (bottom, middle level, top) was characterised by sequencing the products of PCR amplification (as in example 1).

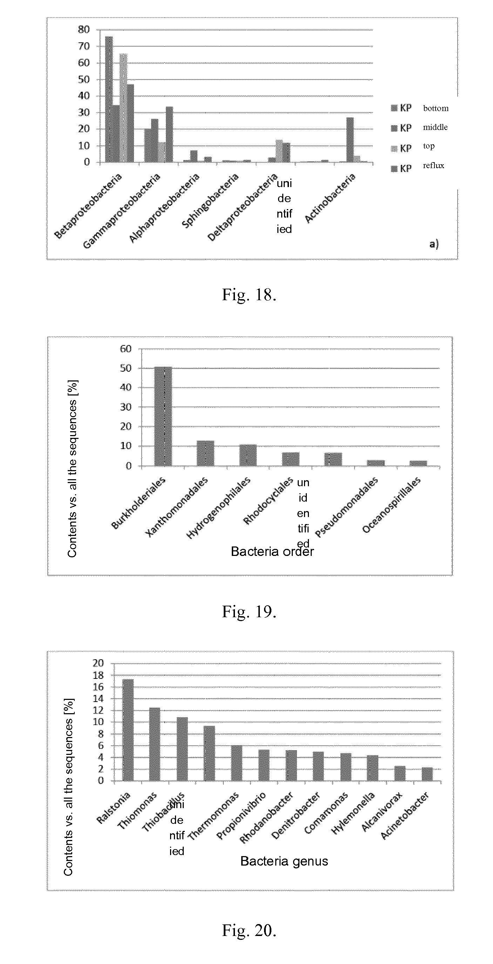

Fig. 18. A list of percentage shares of particular bacteria classes at the three levels of the column packing - the bottom (blue colour), the middle level (red colour) and the top (green colour) and in the reflux (violet colour) on a semi-technical scale at the sewage treatment plant 1.

Fig. 19. Percentage shares of particular bacteria orders at the lower part of the column packing on a semi-technical scale at the sewage treatment plant 1.

Fig. 20. Percentage shares of particular bacterial genera at the lower part of the column packing on a semi-technical scale at the sewage treatment plant 1.

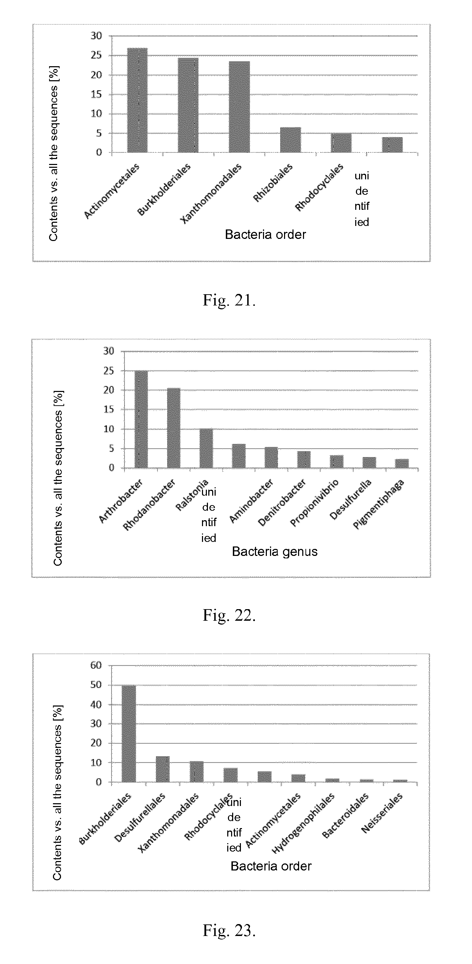

Fig. 21. Percentage shares of particular bacteria orders at the middle part of the column packing on a semi-technical scale at the sewage treatment plant 1.

Fig. 22. Percentage shares of particular bacteria genera at the middle part of the column packing on a semi-technical scale at the sewage treatment plant 1.

Fig. 23. Percentage shares of particular bacteria orders at the upper part of the column packing on a semi-technical scale at the sewage treatment plant 1.

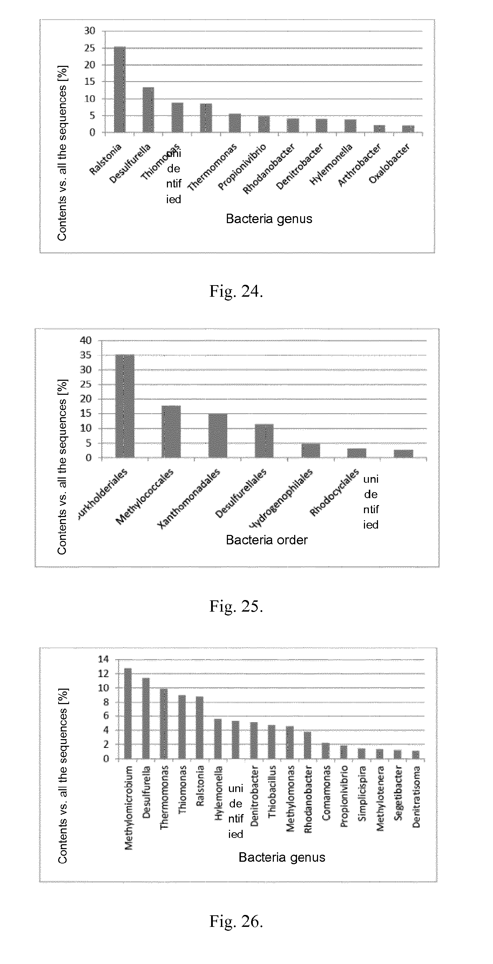

Fig. 24. Percentage shares of particular bacteria genera at the upper part of the column packing on a semi-technical scale at the sewage treatment plant 1.

Fig. 25. Percentage shares of particular bacteria orders in the column reflux on a semi-technical scale at the sewage treatment plant 1.

Fig. 26. Percentage shares of particular bacteria orders in the column reflux on a semi-technical scale at the sewage treatment plant 1.

**Table 3. List of species in the highest number in particular parts of the column packing on a semi-technical scale at the sewage-treatment plant 1..**

| Bacteria species | Localisation in column | | | |
|---|---|---|---|---|
| | the bottom | the middle level | the top | reflux |
| unidentified | 29.75 | 45.46 | 39.76 | 50.99 |
| *Ralstonia insidiosa* | 16.3 | 9.46 | 24.35 | 8.27 |
| *Aminobacter aminovorans* | 0 | 5.2 | 0.29 | 0 |
| *Arthrobacter halodurans* | 0.01 | 4.95 | 0.46 | 0 |
| *Arthrobacter psychrolactophilus* | 0 | 4.33 | 0.49 | 0 |
| *Arthrobacter stackebrandtii* | 0 | 7.92 | 0.77 | 0 |
| *Comamonas composti* | 4.36 | 0.56 | 1.4 | 1.99 |
| *Denitrobacter permanens* | 1.78 | 1.49 | 1.17 | 2.06 |
| *Oxalobacter vibrioformis* | 1.75 | 0.99 | 2.09 | 0.78 |
| *Propionivibrio dicarboxylicus* | 5.32 | 3.72 | 4.76 | 1.84 |
| *Rhodanobacter lindaniclasticus* | 3.8 | 0.41 | 0.63 | 2.61 |
| *Thermomonas dokdonensis* | 2.59 | 0.82 | 2.26 | 3.78 |
| *Thiobacillus thiophilus* | 10.18 | 1.71 | 0.52 | 3.39 |
| *Thiomonas intermedia* | 11.78 | 1.58 | 7.9 | 8.22 |

### Example 3:Microbiological consortium at the column packing on a semi-technical scale at the sewage treatment plant 2.

A biogas desulphurisation system was applied on a semi-technical scale at machanic-biological sewage treatment plant 2, with capacity of 11616 cubic meters / day of raw sewage, both communal and industrial. The sewage treatment plant has got a biological system for nitrogen and phosphorus removal, based on an active sediment. Sediment stabilisation is continued in the process of mesophilic fermentation in two closed chambers, following previous densification of excessive sediments. Desulphurisation efficiency after 15 days of the column operation was 98%. The microbiological consortium at three column levels (bottom, middle level, top) was characterised by sequencing the products of PCR amplification (as in example 1).

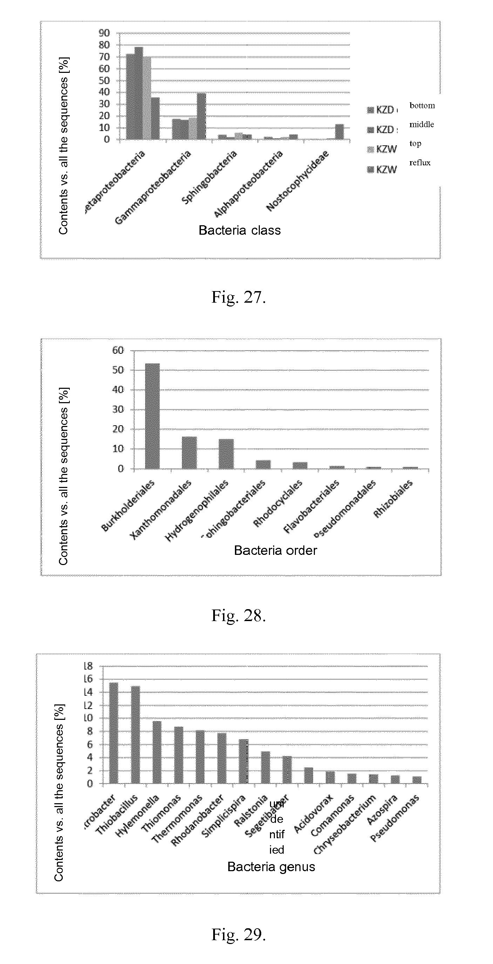

Fig. 27. A list of percentage shares of particular bacteria classes at the three levels of the column packing - the bottom (blue colour), the middle level (red colour) and the top (green colour) and in the reflux (violet colour) on a semi-technical scale at the sewage treatment plant 2.

Fig. 28. Percentage shares of particular bacteria orders at the lower part of the column packing on a semi-technical scale at the sewage treatment plant 2.

Fig. 29. Percentage shares of particular bacterial genera at the lower part of the column packing on a semi-technical scale at the sewage treatment plant 2.

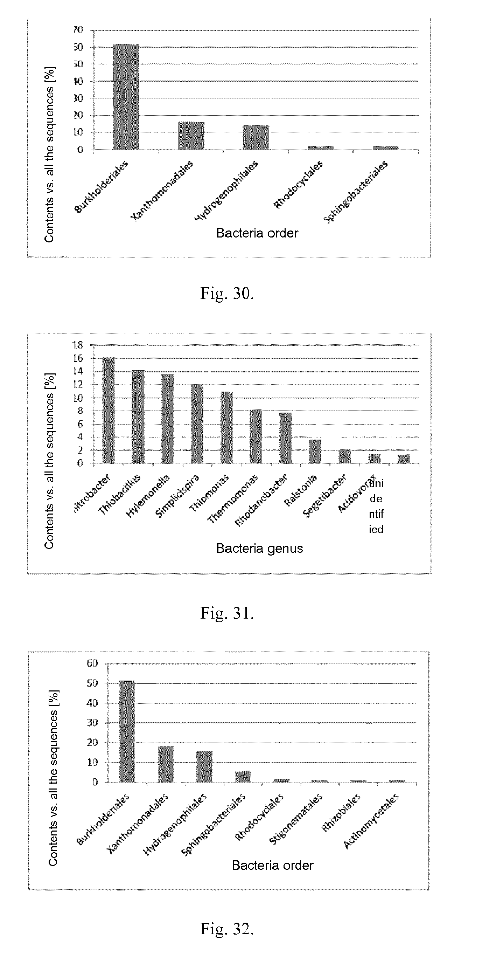

Fig. 30. Percentage shares of particular bacteria orders at the middle part of the column packing on a semi-technical scale at the sewage treatment plant 2.

Fig. 31. Percentage shares of particular bacteria genera at the middle part of the column packing on a semi-technical scale at the sewage treatment plant 2.

Fig. 32. Percentage shares of particular bacteria orders at the upper part of the column packing on a semi-technical scale at the sewage treatment plant 2.

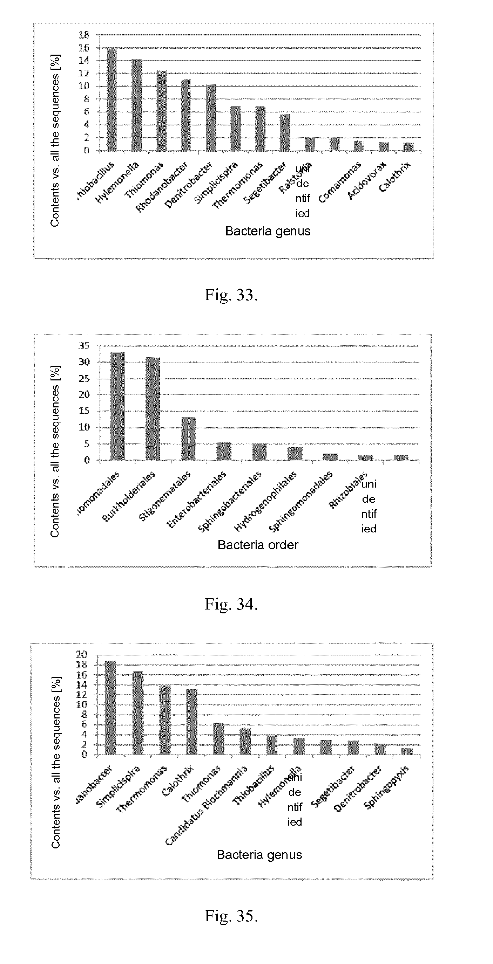

Fig. 33. Percentage shares of particular bacteria genera in the upper part of the column packing on a semi-technical scale at the sewage-treatment plant 2..

Fig. 34. Percentage shares of particular bacteria orders in the column reflux on a semi-technical scale at the sewage treatment plant 2.

Fig. 35. Percentage shares of particular bacteria orders in the column reflux on a semi-technical scale at the sewage treatment plant 2.

**Table 4. List of species in the highest number in particular parts of the column packing on a semi-technical scale at the sewage-treatment plant 2.**

| bacteria species | Localisation in column | | | |
|---|---|---|---|---|
| | the bottom | the middle level | the top | reflux |
| unidentified | 44.08 | 53.81 | 41.71 | 43.68 |
| *Chryseobacterium soli* | 1.35 | 0.36 | 0.76 | 0.01 |
| *Comamonas composti* | 1.03 | 0.65 | 1.21 | 0.22 |
| *Denitrobacter permanens* | 2.66 | 1.57 | 1.93 | 0.32 |
| *Ralstonia insidiosa* | 4.85 | 3.55 | 1.89 | 0.06 |
| *Rhodanobacter lindaniclasticus* | 6.78 | 7.1 | 9.56 | 15.29 |
| *Segetibacter aerophilus* | 4.23 | 2.06 | 5.66 | 2.7 |
| *Thermomonas dokdonensis* | 1.22 | 0.96 | 0.61 | 1.59 |
| *Thiobacillus thiophilus* | 12.97 | 11.79 | 13.79 | 3.43 |
| *Thiomonas intermedia* | 8.21 | 10.63 | 11.68 | 6.06 |

## Claims

1. The microbiological consortium, which purifies biogas in presence of oxygen forms of nitrogen, **characterised in that** it includes bacteria of α-Proteobacteria, β-Proteobacteria and γ-Proteobacteria, belonging to the following orders: Burkholderiales, Xanthomonodales, Rhodocyclales, Hydrogenophilales, Rhizobiales, Sphingomonodales, Enterobacteriales, Methylophilales, Caulobacteriales, Neisseriales, Rhodospirrales, Pseudomonadales, Oceanospirillales, Methylococcales, in particular, belonging to the following genera: Rhodanobacter, Thiomonas, Ralstonia, Thermomonas, Simplicispira, Thiobacillus, Propionivibrio, Sphingomonas, Oxalobacter, Denitratisoma, Thermomonas, Parvibaculum, Simplicispira, Hylemonella , Comamonas , Citrobacter , Pedomicrobium , Methylotenera , Alcanivorax , Acinetobacter , Aminobacter , Methylomicrobium , Methylomonas , Acidovorax , Azospira , Pseudomonas , Candidatus Blochmannia and Sphingopyxis.

2. The microbiological consortium, acc. to claim 1, characteristic in that it contains the following bacteria species: Citrobacter werkmanii , Denitratisoma oestradiolicum , Denitrobacter permanent , Limnohabitans parvus , Luteibacter anthropi , Methylotenera versatilis , Oxalobacter vibrioformis, Propionivibrio dicarboxylicus, Ralstonia insidiosa, Rhodanobacter fulvus , Rhodanobacter ginsenosidimutans , Rhodanobacter lindaniclasticus, Thermomonas dokdonensis, Thermomonas Fusa, Thiobacillus thioparus , Thiobacillus thiophilus , Thiomonas intermedia , Thiomonas perometabolis , Vogesella perlucida , Ralstonia detusculanense , Aminobacter aminovorans , Comamonas composti.

3. The microbiological consortium, acc. to claim 1 or 2, characteristic in that it also includes bacteria, belonging to the following classes: Actinobacteria, Sphingobacteria, Verrucomicrobiae, Holophagae, Planctomycetia, Verrucomicrobiae, Clostridia, ε-Proteobacteria, Δ-Proteobacteria, Flavobacteria, Cyanobacteria, Bacteriodetes which belong to the following orders: Actinomycetales, Sphingobacteriales, Holophagales, Bacteroidales , Verrucomicrobiales , Gemmatales, Campylobacteriales, Desulfurellales, Flavobacteriales, Stigonematales, in particular, belonging to the following genera: Geothrix, Rhodocuccus, Prosthecobacter, Singulisphaera, Cellulomonas, Arthrobacter, Desulfurella, Segetibacter, Chryseobacterium.

4. The microbiological consortium, acc. to claim 3. characteristic in that it includes bacteria of the following specied: Rhodothermus clarus, Arthrobacter halodurans, Arthrobacter psychrolactophilus, Arthrobacter stackebrandtii , Chryseobacterium soli and Segetibacter aerophilus.
